# FASCICULE DE BREVET EUROPEEN

(11) **EP 0 485 577 B1**
(45) Date de publication et mention de la délivrance du brevet: **30.08.1995**
(21) Numéro de dépôt: 91910767.2
(22) Date de dépôt: 06.06.1991
(51) Int. Cl.: A61K 38/00, A61K 38/26, A61K 39/39, A23K 1/16, C07K 9/00

(54) **MEDICAMENT IMMUNOSTIMULANT A BASE DE GLYCOPEPTIDOLIPIDES POLAIRES DE MYCOBACTERIUM CHELONAE**
IMMUNSTIMULIERENDES ARZNEIMITTEL, DAS POLARE GLYCOPEPTIDOLIPIDE VON MYCOBAKTERIUM-CHELONAE ENTHÄLT
IMMUNOSTIMULANT DRUG BASED ON POLAR GLYCOPEPTIDOLIPIDS OF MYCOBACTERIUM CHELONAE

(30) Priorité: 06.06.1990 FR 9006997
(43) Date de publication de la demande: 20.05.1992
(73) Titulaire: NEWAY, Tsehay, F-92260 Fontenay-aux-Roses (FR); PILET, Charles, 94100 St.-Maur (FR)
(72) Inventeur: NEWAY, Tsehay, F-92260 Fontenay-aux-Roses (FR); PILET, Charles, 94100 St.-Maur (FR)
(74) Mandataire: Tonnellier, Jean-Claude
(86) Numéro de dépôt international: FR9100449
(87) Numéro de publication internationale: WO9118617

(56) Documents cités:
- BIOLOGICAL ABSTRACTS, vol. 89, N 1, January 1990, Philadelphia, PA, US; T. NEWAY et al.: "Immunomodulatory properties of a strain of mycobacterium chelonae I. Mouse lymphocyte responses in vitro", p. AB-438-9, Abstract No. 4236
- CHEMICAL ABSTRACTS, vol. 100, N . 17, 23 April 1984, Columbus, Ohio, US; A.Y. TSANG et al.: "Antigenic relationships of the mycobacterium fortuitum-mycobacterium chelonae complex", p. 479, Abstract No. 137060x
- INFECTION AND IMMUNITY, vol. 56, N . 5, May 1988, Washington, DC, US; P.E. BROWNBACKN et al.: "Modified lymphocyte response to mitogens after intraperitoneal injection of glycopeptidolipid antigens from mycobacterium avium complex", pp. 1044-1050

## Description

La présente invention a pour objet un nouveau médicament immunostimulant contenant comme principe actif des glycopeptidolipides polaires (GPLp) de Mycobactérium chelonae.

On sait que les glycopeptidolipides polaires (GPLp), qui sont présents dans la paroi des mycobactéries atypiques, présentent une grande spécificité d'espèce.

Les glycopeptidolipides (GPL) sont des composés associant des acides gras, des peptides et des sucres.

Des études très approfondies de ces GPL ont été réalisées dans un but de classification et d'identification des mycobactéries.

Des auteurs ont montré la diminution de la réponse proliférative, induite par les mitogènes non spécifiques, des splénocytes de souris traitées par les GPLp de M.avium (voir Brownback et Barrow, Infection and Immunity, 56, 1044-1050 (1988)). Les mêmes auteurs ont également montré la diminution de la réponse proliférative de ces cellules lors des costimulations in vitro avec ces GPLp et des mitogènes.

Les propriétés immunostimulantes de M.chelonae vivante sur les cellules immunocompétentes ont été démontrées (voir Biozzi et al., Rev. Franç. Etudes Clin. Biol., 5, 867-890 (1960) ; Pilet et Goret, J. Réticuloendoth. Soc. 3, 305-309 (1966) ; Neway et al., Comp. Immunol. Infect. Dis., 12, 63-70 (1989)).

On a maintenant découvert que les GPLp de M.chelonae possèdent, contrairement aux GPLp de M. avium, des propriétés immunostimulantes.

On sait que les GPL de la plupart des mycobactéries atypiques ont en commun la structure :
Phé-aThr-Ala-Alaninol,
le groupement Phé amino-terminal étant acylé par un acide gras à longue chaîne, le groupement Alaninol étant lié à un sucre, et le groupement allo-thréonine (aThr) étant lie, soit à un sucre (GPL apolaires) soit à un oligosaccharide (GPL polaires). Cette structure glycopeptidolipidique est la même notamment chez toutes les mycobactéries du complexe M.A.I.S. (Mycobacterium Avium-Intracellulare-Scrofulaceum) et chez les deux sous-espèces de M.chelonae (Voir Brennan et Goren, J. Biol. Chem. 254, 4205-4211 (1979) ; Brennan, Rev. Infect. Dis. 3, 905-913 (1981) ; Brennan "The Mycobacteria : A Source book", part A, Kubica and Wayne Eds, Marcel Dekker, New York and Basel, 467-489 (1984) ; et Tsang et al, Int. J. Syst. Bacteriol. 34, 35-44 (1984) ; Asselineau et Asselineau, "The Micobacteria : A Source Book", Part A, Kubica and Wayne Eds, Marcel Dekker, New York and Basel, pp. 345-360, (1984).

Les GPLp de M.chelonae ont été décrits par TSANG et al., article déjà cité. Du fait que les variations d'espèce des GPLp de mycobactéries atypiques concernent la partie osidique, les mêmes auteurs ont étudié cette partie osidique chez M.chelonae et ont proposé pour l'oligosaccharide la structure suivante : 3,4-di-O-méthylrhamnose-(1 --> ?)-rhamnose-(α -1--> 2)-6-désoxytalose.

Les GPLp ont été utilisés dans l'identification et la différenciation de M.chelonae du complexe M.fortuitum-M.chelonae par chromatographie sur couche mince (Tsang et al., article cité) et par la méthode ELISA (Yanagihara et al., J. Clin. Microbiol. 21, 569-574 (1985)).

En définitive, les GPLp de M.chelonae répondent à la formule 1 : 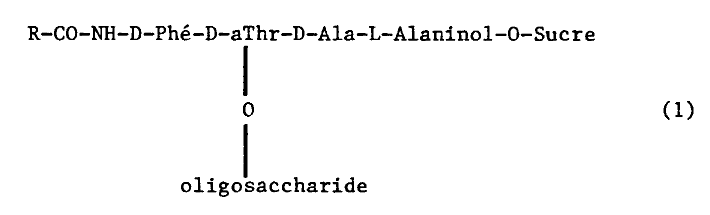
où Phé représente la phénylalanine,
aThr représente l'allo-thréonine,
Ala représente l'alanine,
le sucre est le 3,4-di-O-méthyl rhamnose, l'oligosaccharide a la structure suivante :
3,4-di-O-méthylrhamnose-rhamnose-6-désoxytalose,
R-CO- est le reste acyle d'un acide gras,
les groupements 3,4-di-O-méthylrhamnose étant reliés respectivement à l'allo-thréonine et à l'alaninol par une liaison glycosidique,
et le groupement C terminal de l'alanine étant lié à l'alaninol par une liaison amide.

La structure plus précise de l'oligosaccharide est celle donnée par Tsang et al., comme indiqué ci-dessus, dans laquelle cependant la position de la liaison du rhamnose au diméthylrhamnose n'est pas connue.

En outre, les sucres de la partie oligosaccharidique sont acétylés dans les GPLp naturels..

Dans les GPLp de M.chelonae, de même que dans les GPL des autres Mycobactéries, la nature des acides gras (longueur de chaîne, présence éventuelle d'une ou plusieurs doubles liaisons, d'une ramification ou d'un substituant bêta-hydroxy ou bêta-méthoxy) dépend du milieu de culture, de la température et de la durée de la culture ; voir par exemple Ratledge, "Lipids : Cell Composition, Fatty Acid Biosyntheses", in "The Biology of the Mycobacteria", Vol. 1, Ratledge and Stanford Eds, Academic Press, London (1982) pages 53-93, et les articles ou ouvrages déjà cités précédemment.

Les GPLp naturels sont en fait des mélanges de composés de formule 1, dans lesquels le groupement acyle gras R-CO- est variable. Ces acides gras ont au moins 16 atomes de carbone, et ont généralement moins de 36 atomes de carbone.

La présente invention a donc pour objet une composition pharmaceutique immunostimulante contenant comme principe actif au moins un GPLp de M.chelonae, ou un dérivé, actif comme immunostimulant, de GPLp de M.chelonae.

Les GPLp présents dans la composition pharmaceutique de l'invention sont notamment des fractions d'extraction, au moins partiellement purifiées, des glycopeptidolipides de la paroi de M.chelonae, obtenues selon les méthodes connues, et il est bien entendu que l'invention ne s'étend pas à des compositions pharmaceutiques contenant comme seule source de GPLp de M.chelonae des M.chelonae vivantes ou tuées, ou des parois entières ou fragments de parois de ces mycobactéries.

Les fractions d'extraction des GPLp au moins partiellement purifiées utilisées comme principe actif dans les compositions pharmaceutiques de l'invention sont notamment des extraits glycopeptidolipidiques de la paroi de M.chelonae qui sont solubles par exemple dans le méthanol froid (en particulier dans le méthanol à + 4°C).

Ce sont en particulier des fractions glycopeptidolipidiques qui peuvent être obtenues par extraction de cellules ou de parois de M.chelonae à l'aide d'un mélange chloroforme : méthanol (2 : 1) à une température de 18 à 50 °C, et qui, en outre, sont solubles à froid dans le méthanol, par exemple qui restent solubles après addition de méthanol froid (4°C) en quantité suffisante pour que le rapport méthanol : chloroforme (en volume) soit au moins égal à 5 : 1. Ces fractions peuvent également être obtenues par extraction comme indiqué ci-dessus puis purifiées par chromatographie sur colonne de gel de silice.

L'invention a en particulier pour objet une composition pharmaceutique immunostimulante contenant comme ingrédient actif au moins un composé de formule 1, ou un dérivé actif de celui-ci.

Les GPLp présents dans les compositions pharmaceutiques de l'invention peuvent être non seulement les GPLp naturels acétylés, mais aussi les dérivés actifs de GPLp, et en particulier les dérivés désacétylés correspondants.

Les GPLp ou dérivés de GPLp constituant le principe actif du médicament de l'invention peuvent être préparés à partir de souches de M.chelonae.

Des souches de M.chelonae peuvent être obtenues notamment auprès des collections publiques suivantes :
- NCTC-National Collection of Type Cultures (U.K.) n° de dépôt 946 (M.chelonae sous-espèce chelonae)
- ATCC-American Type Culture Collection (U.S.A.) n° de dépôt 19977 (M.chelonae sous-espèce abscessus)
- CIPT-Collection Institut Pasteur Tuberculose, Paris (France) n° de dépôt 140420019 (M.chelonae sous-espèce chelonae)
- CIPT-Collection Institut Pasteur Tuberculose, Paris (France) n° de dépôt 140420020 (M.chelonae sous-espèce abscessus)
M.chelonae peut être entretenu en milieu de Lowenstein-Jensen (Institut Pasteur-Paris) et cultivé en milieu de Sauton mis dans des boites de Roux après solidification par addition de 1,5 % de Bacto-agar (DIFCO). M.chelonae peut également être cultivée en fermenteurs ou à l'aide de cultures en voiles ou de tous autres moyens similaires.

L'extraction des GPLp de M.chelonae est connue, comme mentionné ci-dessus. Elle consiste à extraire les complexes lipidiques totaux de la bactérie vivante ou tuée ou de la paroi bactérienne. On effectue par exemple l'extraction à l'aide de mélanges chloroforme-méthanol, sur les bactéries, y compris sur les bactéries lyophilisées.

On peut éliminer ensuite, au moins partiellement, les glycolipides, les carotènoïdes et les lipides libres. On peut par exemple opérer par addition de quantités importantes de méthanol, à froid, dans une solution des complexes lipidiques totaux dans le chloroforme. Il y a alors précipitation des glycolipides, des carotènoïdes et de lipides libres. Les GPL polaires et apolaires, les phospholipides et les autres lipides libres restent en solution.

Il est intéressant à ce stade de procéder, de façon connue, à une désacétylation par traitement alcalin modéré (addition d'une solution de NaOH dans le méthanol). Aux complexes lipidiques issus de l'étape ci-dessus, en solution dans un mélange chloroforme-méthanol, en particulier un mélange chloroforme-méthanol (2 : 1), on ajoute par exemple un volume égal d'une solution 0,2M de NaOH dans le méthanol. Cette désacétylation est un caractère spécifique des GPL des mycobactéries qui résistent à ce traitement alcalin alors que les autres lipides que l'on souhaite éliminer (phospholipides, carotènoïdes et d'autres lipides indésirables) sont dégradés. La désacétylation facilite aussi leur séparation et leur purification ultérieures en chromatographie sur colonne et/ou sur couche mince. Après le traitement alcalin, on neutralise avec un acide, par exemple l'acide acétique concentré. Il est avantageux, à ce stade, de laver la phase organique avec un mélange de chloroforme : méthanol : eau, 4 : 2 : 1, ou un mélange similaire. La phase organique lavée peut ensuite être soumise à une purification par chromatographie sur colonne soit par la méthode de TSANG et al., article déjà cité, soit par la méthode de Dimitrijevich et al., J. Chromato., 377, 345-349 (1986).

On obtient ainsi une fraction partiellement purifiée contenant les GPLp de M.chelonae.

La chromatographie sur colonne permet de séparer les lipides indésirables échappant à la destruction par le traitement alcalin, les glycopeptidolipides apolaires, les glycopeptidolipides polaires, et éventuellement les glycolipides contenant du tréhalose (qui sont présents dans le cas où la précipitation au méthanol froid n'a pas été réalisée).

Les glycopeptidolipides apolaires de M.chelonae sont des fractions proches du front du solvant et qui se colorent en jaune rosé à l'orcinol dans l'épreuve de chromatographie sur couche mince ; ils ne sont pas spécifiques de l'espèce (voir par exemple Tsang et coll., article cité).

Les glycopeptidolipides polaires de M.chelonae, qui sont spécifiques de l'espèce, sont des fractions éloignées du front du solvant et qui se colorent en marron doré chez M.chelonae sous-espèce chelonae, et en orange foncé chez M.chelonae sous-espèce abscessus, dans l'épreuve à l'orcinol en chromatographie sur couche mince, (voir par exemple Tsang et al., article cité).

L'ingrédient actif des compositions de l'invention peut aussi être au moins un composé de formule 1 obtenu, par synthèse ou hémisynthèse, selon les techniques classiques de la synthèse peptidique et saccharidique.

La composition de l'invention est préparée selon les méthodes galéniques usuelles.

Les GPLp, ou les fractions d'extraction les contenant, peuvent être soumis à l'action des ultrasons pendant 20 à 50 minutes, avec une longueur d'onde de 8 »m, à une concentration de GPLp de 1 à 20 mg/ml, afin d'homogénéiser le produit, dans un véhicule liquide approprié.

Dans les compositions de l'invention, l'ingrédient actif (GPLp) est généralement présent à raison de 0,02 à 8 % en poids, par rapport au poids de la composition.

Les compositions de l'invention peuvent se présenter notamment sous la forme d'une suspension dans un véhicule pharmaceutique liquide convenable. La suspension peut contenir un agent tensioactif utilisable en pharmacie, en particulier un tensioactif non ionique tel qu'un mono-oléate de sorbitan polyoxyéthyléné (par exemple le Tween® 80). Les compositions liquides peuvent être soumises à une lyophilisation, éventuellement avec un adjuvant de lyophilisation, conservées sous forme de lyophilisat, et reconstituées au moment de l'emploi. La composition liquide prête à l'emploi contient généralement de 0,2 à 80 mg/ml de GPLp (en fonction de la voie d'administration utilisée).

La suspension peut également contenir jusqu'à 0,2 % en volume de tensioactif. Les compositions de l'invention peuvent généralement contenir une quantité efficace d'un agent conservateur usuel, par exemple le merthiolate.

Les compositions de l'invention sont notamment des compositions conditionnées sous la forme de suspensions buvables ou injectables, ou pour application locale, ou encore sous la forme de compositions conditionnées pour l'application par voie nasale ou conjonctivale.

Les compositions de l'invention peuvent être également présentées sous forme de gélules, de comprimés, de poudres, de suppositoires, de pâtes gingivales ou de crèmes.

Comme on le verra dans la partie expérimentale ci-après, les GPLp de M.chelonae sont des stimulants des réponses de défense non spécifique de l'organisme. Ils peuvent également stimuler de façon non spécifique une réaction immunitaire spécifique (effet adjuvant).

La composition de l'invention est donc utilisable comme médicament immunostimulant, chez l'homme ou chez l'animal. Elle est également utilisable notamment comme adjuvant de l'immunité conférée par les vaccins et comme potentialisateur d'antibiothérapie.

La composition de l'invention peut être également utilisée comme facteur de croissance et/ou anabolisant chez les animaux et chez les humains.

Elle peut être administrée notamment par voie parentérale (intra-péritonéale, sous-cutanée, intra-musculaire, intraveineuse, percutanée), par voie orale, par voie nasale, par voie conjonctivale, par voie rectale ou par voie per-linguale.

Elle peut aussi être utilisée en application locale, à l'aide de pâtes gingivales ou de comprimés à délitement buccal, notamment dans l'immunothérapie non spécifique des maladies de la cavité buccale (pyorrhée alvéolo-dentaire, ginivites, parodontites, etc.).

La posologie habituelle peut aller par exemple de 0,1 à 12, en particulier de 0,5 à 10 mg/kg de poids corporel et par jour, en une ou plusieurs administrations. Par exemple, elle est le plus souvent de 0,5 à 3 mg/kg pour la voie parentérale, de 4 à 10 mg/kg pour la voie orale, et de 2 à 4 mg/kg pour la voie nasale.

Le médicament de l'invention est administré, à dose efficace, notamment à titre de traitement immunostimulant, dans les cas de déficit immunitaire secondaire ; comme traitement adjuvant dans les cas de maladies infectieuses locales ou générales ; comme traitement adjuvant dans le traitement du syndrome immunodéficitaire acquis, notamment en association avec des traitements antiviraux ; comme traitement adjuvant dans les cas de maladies parasitaires et d'atteintes cancéreuses ; et comme traitement correcteur des effets immunodépresseurs (révélés notamment par la leucopénie) des thérapeutiques anti-cancéreuses.

Le médicament de l'invention peut être administré également à titre prophylactique, dans les différents cas ci-dessus, et notamment pour la prévention des infections récidivantes de la sphère otorhinolaryngologique, et pour la prévention des risques infectieux chez les malades chroniques, ainsi que comme adjuvant de vaccins.

Il peut également être utilisé pour favoriser la croissance ou la prise de poids chez les animaux et chez les humains.

Les GPLp de M.chelonae peuvent en particulier être utilisés comme additifs alimentaires pour animaux, destinés à favoriser la croissance et/ou la résistance à l'infection.

L'invention a également pour objet l'utilisation des GPLp de M.chelonae, tels que définis ci-dessus, comme ingrédients actifs dans la préparation d'un médicament immunostimulant non spécifique, ou d'un médicament anabolisant, comme adjuvants dans la réalisation d'un vaccin, ou encore comme additifs dans la préparation d'une composition alimentaire.

L'invention concerne en particulier l'utilisation des GPLp de M-chelonae comme ingrédients actifs dans la préparation d'un médicament immunostimulant non spécifique destiné notamment à corriger les effets immunodépresseurs (par exemple les leucopénies chimio-induites) des thérapeutiques anti-cancéreuses.

Les exemples suivants illustrent l'invention sans toutefois la limiter.

### EXEMPLE 1

### PURIFICATION D'UNE FRACTION GPLp DE M.CHELONAE

### CULTURE DES BACTERIES

Mycobacterium chelonae sous-espèce chelonae est cultivée à 35°C dans les milieux qui ont été indiqués dans la description ci-dessus, jusqu'à la phase stationnaire, puis récoltée et lavée par centrifugation à 5900 x g. Les cellules sont ensuite séchées ou lyophilisées et soumises à l'extraction dans les plus brefs délais.

### EXTRACTION DES COMPOSES LIPIDIQUES TOTAUX :

L'extraction des lipides totaux de la paroi de M.chelonae est réalisé selon une technique voisine de celle décrite par Brennan et Goren, Journal of Biological Chemistry, Vol. 254, n° 10, 4205-4211 (1979).

On procède à une extraction à l'aide d'un mélange chloroforme : méthanol (2 : 1), à raison de 40 ml du mélange par gramme de mycobactéries lyophilisées, à 50 °C pendant 18 heures dans un ballon immergé dans un bain-marie. L'extrait est ensuite récupéré par filtration sur papier Whatman n° 3. On soumet le résidu à une nouvelle extraction, de la même façon, mais pendant 4 heures seulement. On réunit les extraits et on évapore les solvants. L'extrait sec est conservé à + 4 °C jusqu'à l'étape suivante.

### ELIMINATION DES GLYCOLIPIDES, DES CAROTENOIDES ET DES LIPIDES LIBRES

Les complexes lipidiques totaux sont solubilisés avec une quantité suffisante de chloroforme et on ajoute une quantité importante de méthanol à +4°C. On observe alors une précipitation. La quantité de méthanol doit être suffisante pour qu'une addition ultérieure de méthanol ne provoque plus de précipitation. Le précipité contient des glycolipides, des carotènoïdes et des lipides libres. Les GPLp, les GPL apolaires, les phospholipides et les autres lipides libres restent en solution.

On élimine le précipité par centrifugation, puis on évapore les solvants du surnageant.

### DESACETYLATION ET LAVAGE :

On soumet le résidu d'évaporation obtenu au stade précédent à un traitement alcalin modéré (désacétylation), et on effectue ensuite un lavage avec un mélange chloroforme-méthanol-eau.

Pour cela, le résidu obtenu au stade précédent est repris dans un mélange chloroforme : méthanol (2 : 1), et on ajoute un même volume d'une solution de NaOH 0,2M dans le méthanol. Après 30 minutes à 37 °C le mélange est neutralisé par 12,5 »l/ml d'acide acétique concentré, puis lavé. Pour cela, on ajuste la proportion du mélange chloroforme : méthanol : eau à 4 : 2 : 1. Après agitation et libération du gaz qui se produit, on laisse le mélange au repos pendant 1 heure. La phase aqueuse est éliminée et la phase organique est recueillie et soumise à l'évaporation. Le résidu est conservé à + 4°C.

### SEPARATION ET PURIFICATION DES GPL EN CHROMATOGRAPHIE SUR COLONNE DE GEL DE SILICE

On prépare une colonne en verre contenant 100 g de gel de silice 60 (granulométrie 0,063-0,200 mm) par g de complexe lipidique. On opère selon la méthode décrite par Tsang et al., article cité, qui consiste à séparer les constituants avec un pourcentage croissant de méthanol dans le chloroforme, en commençant avec le chloroforme pur. On peut également opérer selon la méthode de Dimitrijevich et coll. (article cité), qui chromatographient avec un pourcentage fixe de méthanol (10 %) dans le chloroforme. Le débit est fixé à 1,5 ml/min environ. Les fractions sont analysées par chromatographie analytique sur couche mince. Les fractions de mêmes mobilités sont réunies, pesées et conservées, de préférence à 4 °C.

### CHROMATOGRAPHIE SUR COUCHE MINCE (CCM)

On utilise des plaques de CCM (plaques de verres 20 x 20 cm et de 1 mm d'épaisseur) (Merck) activées par chauffage à 110°C pendant 30 minutes avant l'utilisation.

Les fractions obtenues au stade précédent sont ajustées à 10 mg/ml dans le mélange chloroforme : méthanol (2 : 1). Ces préparations sont alors déposées en taches équidistantes (minimum 1 cm) de un à deux centimètres du bord inférieur de la plaque. Ces dépôts sont réalisés à l'aide de pipettes Pasteur effilées et courbées à leur extrémité. Les plaques sont alors déposées dans une cuve contenant le solvant de migration (chloroforme : méthanol : eau / 60 : 12 : 1). Lorsque le solvant a migré jusqu'à 1 ou 2 cm du bord supérieur de la plaque, la migration est interrompue. On sèche la plaque sous une hotte ventilée, et on pulvérise le réactif de révélation. La révélation des GPLp est faite par pulvérisation de 0,1 % d'orcinol dans une solution d'acide sulfurique à 40 % dans de l'eau bidistillée. Après passage pendant 3 à 10 minutes dans un four à une température de 110 à 130 °C les GPLp spécifiques de M.chelonae sont colorés en marron doré alors que les glycopeptidolipides apolaires sont colorés en jaune rosé. Cette chromatographie permet de contrôler le degré de pureté de l'extrait séparé.

Pour l'identification et le contrôle de purification des GPLp, les méthodes analytiques suivantes peuvent être utilisées :

### HYDROLYSE ACIDE AVEC HCl 1N

L'hydrolyse acide des GPL spécifiques de M.chelonae en présence de HCl 1N permet la libération des sucres spécifiques (méthylrhamnose, rhamnose et désoxytalose) que l'on peut identifier par chromatographie sur papier Whatman n° 1 et par chromatographie en phase gazeuse.

### HYDROLYSE ACIDE AVEC HCl 6N

L'hydrolyse acide avec HCl 6N du résidu de l'étape précédente permet de libérer les acides aminés spécifiques des GPL des mycobactéries, qui peuvent être identifiés par chromatographie sur couche mince. Dans le cas de M.chelonae, comme dans le cas des mycobactéries atypiques, ces acides aminés sont la phénylalanine, l'alanine, l'alaninol et l'allothréonine.

### ANALYSE DES GPLp PAR LE SPECTRE INFRA ROUGE

L'analyse des GPLp désacétylés de M.chelonae a montré des pics caractéristiques des liaisons peptidiques des glycopeptidolipides, analogues à ceux décrits par Brennan et Goren (1979, article cité) et par Brennan (1984, ouvrage cité).

De façon analogue à celle décrite ci-dessus, on a préparé des GPLp purifiés à partir de M.chelonae, sous-espèce abscessus.

### EXEMPLE 2

### PREPARATION D'UNE SUSPENSION INJECTABLE

Les GPLp obtenus à l'exemple 1 sont repris par le chloroforme et évaporés deux fois sous atmosphère d'azote dans un tube de traitement aux ultrasons.

Le résidu est mis en suspension dans un liquide convenant pour la préparation des solutions injectables, par exemple du sérum physiologique (solution apyrogène de NaCl à 8,5 %). On peut également ajouter un détergent compatible avec l'administration parentérale, par exemple le Tween-80, à raison de 0,1 à 0,2 % en volume. L'addition de ce détergent a pour but de faciliter la mise en suspension.

On soumet alors la suspension obtenue à un traitement aux ultrasons pendant environ 20 à 50 min, à une amplitude de 8 »m pour une concentration de 1 à 20 mg de GPLp/ml afin d'homogénéiser le produit.

On peut également préparer des suspensions injectables avec d'autres milieux acceptables tels que le PBS ou analogues.

La concentration des GPLp est ajustée par exemple entre 0,2 et 80 mg/ml, selon les applications. Pour une conservation prolongée, cette préparation peut être lyophilisée, et reconstituée ensuite dans le sérum physiologique sans perte appréciable d'activité.

### EXEMPLE 3

### ETUDE PHARMACOLOGIQUE DES GPLp DU M.CHELONAE

Cette étude a été effectuée avec les GPLp obtenus à l'exemple 1.

### 1. Effet stimulateur de la transformation lymphoblastique

Cet effet est estimé par l'augmentation de la transformation lymphoblastique des splénocytes et des thymocytes de souris préalablement traitées avec le GPLp de M.chelonae par effet direct sur ces cellules, ou, par effet indirect (augmentation de la réponse proliférative aux mitogènes). Ces effets sont mesurés par l'étude de l'incorporation de la thymidine tritiée.

L'étude est effectuée sur des souris femelles Balb/c
Le produit est administré à la dose de 12 mg/kg par voie orale ou de 2,5 mg/kg par voie sous-cutanée (s/c), en 3 administrations à 3 jours d'intervalle (s/c) et 4 administrations à 3 jours d'intervalle (voie orale), sur des lots de 5 animaux. Les lots témoins sont traités avec le solvant.

### Résultats :

Le GPLp stimule la transformation lymphoblastique des splénocytes murins lors des traitements sous-cutanés (p ≦ 0,001) et oraux (p ≦ 0,05).

La réponse aux mitogènes des splénocytes murins et augmentés lors d'une stimulation sous-cutanée : (p ≦ 0,02) pour la Concanavaline A(ConA ; IBF-LKB) ; p ≦ 0,01 pour la Phytohémagglutinine (PHA ; Difco) ; et p ≦ 0,001 pour le lipopolysaccharide B (LPS, Difco). Cette augmentation est également observée lors d'une stimulation orale (p ≦ 0,001 pour la ConA, la PHA et le LPS).

La réponse aux mitogènes des thymocytes murins est également augmentée de façon très significative (p ≦ 0,001 pour la ConA et le LPS).

### 2. Capacité d'induction des monokines par les macrophages péritonéaux de souris Balb/c traitées

Lorsque les macrophages sont mis en contact avec un antigène ou un mitogène possédant des effets immunostimulants, ils répondent par une sécrétion de monokines (interleukine 1, Tumor Necrosis Factor (TNF)). Le test d'induction de monokines par les macrophages péritonéaux est donc un moyen d'évaluation des effets des produits immunostimulants sur ces cellules.

### Résultats :

L'activation, avec le LPS, des macrophages péritonéaux, issus de souris préalablement traitées avec le GPLp a montré une induction très significative d'une activité interleukine 1 (IL-1) (équivalente à 107 unités/ml) sur la transformation des thymocytes des souris C3H/Hej (souris non-sensible au LPS).

Le surnageant de ces macrophages a également présenté un effet toxique sur la lignée tumorale L929 qui est sensible à l'action du TNF (une quantité équivalente à 20000 unités/ml).

La stimulation intra-péritonéale de souris BALB/C par 3 administrations à 3 jours d'intervalle à la dose de 2,5 mg/kg du GPLp a présenté une induction significative de l'activité TNF dans le sérum de ces animaux évalué par la toxicité sur la lignée tumorale L929 (une quantité équivalente à 34000 unités/ml).

### 3. Capacité d'induction de lymphokines par les splénocytes des souris Balb/c

Lorsque les splénocytes sont mis en contact avec un antigène ou un mitogène immunostimulant in vivo ou in vitro, ils répondent par une sécrétion des lymphokines.

La plus connue de ces lymphokines est l'interleukine-2 (IL-2) qui est indispensable pour la survie et la multiplication d'une lignée de lymphocytes T cytotoxiques (Thymome murin) IL-2 dépendante (CTLL-2).

### Résultats :

La stimulation sous-cutanée, avec le GPLp, des souris Balb/c par 3 administrations à 3 jours d'intervalle à la dose de 2,5 mg/kg a présenté une induction significative de l'activité IL-2 mesurée dans le surnageant des cellules spléniques au bout de 48 heures (quantité équivalente à 5,55 unités/ml).

### 4. Tests d'hypersensibilité retardée chez la souris

La réaction d'hypersensibilité retardée (HSR) est déclenchée par un allergène injecté localement (coussinets plantaires) après une première sensibilisation par voie intra-veineuse. Lorsque l'animal est traité avec un immunostimulant non spécifique agissant sur les lymphocytes T, on observe une augmentation de l'hypersensibilité retardée lors d'une deuxième introduction locale de l'allergène.

Dans ce test, on analyse l'augmentation du volume des coussinets plantaires après la deuxième injection d'allergène. On utilise les globules rouges de mouton comme allergène dans cette étude. L'injection par voie intraveineuse des globules rouges de mouton a été réalisée deux jours après la dernière stimulation des souris par voie i/p ou s/c du produit étudié et quatre jours avant l'épreuve d'hypersensibilité retardée. Les souris stimulées reçoivent le produit étudié aux doses de 2,5 mg/kg aux jours J-8, J-5 et J-2 par voie intrapéritonéale ou sous-cutanée. Les souris témoins reçoivent seulement du sérum physiologique. Au jour J+0 toutes les souris reçoivent par voie intra-veineuse une dose unique de 10⁶ globules rouges de mouton. Au jour J+4 les souris reçoivent 10⁸ globules rouges de mouton par voie intra-plantaire.

### Résultats :

La stimulation intra-péritonéale et sous-cutanée de souris C57BL/6 par 3 administrations à 3 jours d'intervalle, à l'aide du produit, a provoqué une augmentation de l'hypersensibilité retardée (p ≦ 0,001) par rapport aux souris témoins (sauf pour la lecture à la 72ème heure) comparable à celle du BCG.

### 5. Augmentation de la réponse anticorps anti-globules rouges de mouton chez la souris

L'ampleur de la réponse en anticorps dépend de la nature de l'antigène et du système immunitaire. Lorsque ce dernier est stimulé à l'aide d'un immunostimulant, ou par un antigène en présence d'un adjuvant, la réponse en anticorps est augmentée. Dans les essais réalisés, cette propriété du GPLp est analysée par la réponse en anticorps anti-globules rouges de mouton, chez les souris C57BL/6 préalablement stimulées par le produit étudié. Les souris traitées reçoivent le produit étudié aux doses 2,5 mg/kg aux jours J-8, J-5 et J-2 par voie intra-péritonéale ou sous-cutanée. Les souris témoins reçoivent seulement du sérum physiologique.

Au jour J+0 toutes les souris reçoivent par voie intra-veineuse une dose unique de 10⁶ globules rouges de mouton. Les sérums prélevés du jour J+0 aux jours J+43 ont été testés pour une capacité d'hémolyse des globules rouges de mouton.

### Résultats:

La stimulation intra-péritonéale et sous-cutanée, par le GPLp de souris C57BL/6 en 3 administrations à 3 jours d'intervalle a présenté une augmentation très significative de la réponse en anticorps anti-globules rouges de mouton pour les prélèvements aux jours 7, 11 et 25 pour la stimulation par voie intra-péritonéale et aux jours 7, 20 et 25 pour la stimulation par voie sous-cutanée.

### 6. Tests de protection globale

### a/ Essais de protection contre une infection à Klebsiella pneumoniae chez la souris :

L'injection par voie intrapéritonéale de K. pneumoniae spécialement adaptée à la souris entraîne une septicémie mortelle en 24 à 48 heures.

L'étude consiste à déterminer le temps de survie des souris traitées par rapport aux témoins. Les souris traitées reçoivent le produit étudié aux doses de 2,5 mg/kg aux jours J-8, J-5 et J-2 par voie intra-péritonéale. Les souris témoins reçoivent seulement du sérum physiologique. Au jour J+0 toutes les souris reçoivent par voie intra-péritonéale une dose appropriée de K.pneumoniae. La mortalité est ensuite observée pendant 5 jours.

### Résultats :

La stimulation intra-péritonéale avec le GPLp par 3 administrations à 3 jours d'intervalle à la dose de 2,5 mg/kg a protégé de façon très significative les souris CD1 infectées par 50xDL50 de K.pneumoniae. Par ailleurs, ces animaux n'ont pas montré de signe de maladie durant l'épreuve.

### b/ Effets de protection contre la leucémie induite par injection de cellules L 1210 chez la souris.

L'étude consiste à déterminer le temps de survie des souris B6D2/F1 traitées par rapport aux témoins.

Les cellules leucémiques L-1210 ont été cultivées en milieu approprié avant l'épreuve.

Les souris traitées reçoivent le produit étudié aux doses de 2,5 mg/kg au jours J-8, J-5 et J-2 par voie intrapéritonéale. Les souris témoins reçoivent seulement du sérum physiologique. Au jour J0 toutes les souris reçoivent par voie intrapéritonéale une dose unique de 10⁶ cellules L 1210 viables. On détermine les temps de survie.

### Résultats :

La stimulation intra-péritonéale avec le GPLp par 3 administrations à 3 jours d'intervalle à la dose de 2,5 mg/kg a prolongé d'une façon très significative le temps de survie des souris B6D2/F1 contre les cellules leucémiques L-1210 (avec une prolongation de survie de 33 % par rapport aux témoins).

### 7. Evaluation de l'effet anabolisant chez la souris par voie s-c

Le produit est injecté par voie s-c à la dose d'environ 2,5 mg/kg en 3 administrations à intervalles de 3 jours. Le poids des souris est enregistré quotidiennement pendant 10 jours et le gain de poids total a été évalué.

Les souris traitées reçoivent le GPLp aux doses de 2,5 mg/kg aux jours J-10, J-7 et J-4. Les souris témoins reçoivent seulement du sérum physiologique.

### Résultats :

La stimulation sous-cutanée avec le GPLp par 3 administrations à 3 jours d'intervalle à la dose de 2,5 mg/kg présente chez la souris BALB/C une propriété anabolisante significative (p ≦ 0,02).

### 8. Effet anti-leucopéniant du GPLp lors de leucopénies chimio-induites

Le test consiste dans un premier temps à induire chez les souris une déplétion en globules blancs à l'aide de l'Adriblastine (doxorubicine chlorhydrate lactose ; produit cytostatique du groupe des anthracyclines). Dans un second temps, ces souris sont traitées par le GPLp et d'autres produits utilisés comme témoins positifs (GM-CSF-souris-Genzyme) jusqu'à la restauration de ces cellules sanguines à la valeur normale.

Les souris Balb/c sont traitées par l'Adriblastine à raison de 5 mg/kg/J par voie intraveineuse aux jours J+0 et J+1 sous un volume de 50 »l. Ces souris reçoivent ensuite le GPLp, le GM-CSF ou le sérum physiologique par voie intra-péritonéale sous un volume de 0,15 ml. Le traitement est administré aux jours J+2, J+5, J+8, J+11, J+14, J+17 et J+20. Les numérations des leucocytes circulants ont été réalisées sur chaque animal aux jours J+0, J+2, J+5, J+8, J+11, J+14, J+17, J+20 et J+28.

### Résultats :

La stimulation intra-péritonéale avec le GPLp des souris BALB/C reconstitue la leucopénie induite par le traitement préalable avec l'Adriblastine de façon très significative, sensiblement camparable à celle de GM-CSF souris.

### 9. Etude de la fonction phagocytaire : Activité du GPLp sur la clearance du carbone colloïdal :

Ce test permet d'étudier la cinétique de l'épuration sanguine de particules de carbone colloïdal par les cellules phagocytaires Les souris traitées reçoivent le produit étudié aux doses de 12 mg/kg aux jours J-8, J-5 et J-2 par voie orale. Les souris témoins reçoivent seulement du sérum physiologique. Au jour J+0 toutes les souris reçoivent par voie intra-veineuse une dose unique de carbone colloïdal (Encre de Chine Pelikan noire) en suspension dans de la gélatine à 4 %, à la dose de 16 mg/100 g de poids vif. Les prélèvements de sang (0,025 ml) sont effectués aux temps 0, 2, 4, 6, 8, 10 et 12 minutes. La lecture des prélèvements s'effectue au spectrophotomètre, longueur d'onde 630. Elle permet de mesurer les particules de carbone restant en suspension dans le sang.

### Résultats :

La stimulation orale avec le GPLp à la dose de 12 mg/kg aux jours J-8, J-5 et J-2 des souris CDI induit une augmentation significative de la clearance du carbone colloïdal.

## Revendications (Revendications pour l'(les) Etat(s) contractant(s) suivant(s): AT, BE, CH, DE, DK, FR, GB, IT, LI, LU, NL, SE)

1. Composition pharmaceutique caractérisée par le fait qu'elle contient comme ingrédient actif une fraction d'extraction au moins partiellement purifiée de glycopeptidolipide (GPL) polaires de Mycobacterium chelonae (M.chelonae) ou d'un dérivé, actif comme immunostimulant, de GPL polaires de M.chelonae.

2. Composition selon la revendication 1, caractérisée par le fait que ladite fraction au moins partiellement purifiée est une fraction soluble dans le méthanol à + 4°C.

3. Composition selon la revendication 1, caractérisée par le fait que ladite fraction est une fraction qui peut être obtenue par extraction de cellules ou de parois de M.chelonae à l'aide d'un mélange chloroforme : méthanol (2 : 1) à une température de 18 à 50 °C, et qui en outre est soluble dans le méthanol à 4 °C.

4. Composition selon la revendication 3, caractérisée par le fait que ladite fraction peut être obtenue par extraction à l'aide d'un mélange chloroforme : méthanol 2 : 1, puis addition de méthanol, refroidi à 4°C, en quantité suffisante pour que le rapport méthanol : chloroforme, en volume, soit au moins égal à 5 : 1, et recueil de la fraction restant en solution.

5. Composition selon la revendication 4, caractérisée par le fait qu'en outre ladite fraction restant en solution est reprise, après évaporation des solvants, dans un mélange chloroforme : méthanol 2 : 1, est soumise, de façon connue, à une désacétylation par addition d'une solution 0,2 M de NaOH dans le méthanol, et qu'après 30 minutes à 37°C, on neutralise avec de l'acide acétique, on ajuste les proportions de solvants pour obtenir un mélange chloroforme : méthanol : eau à 4 : 2 : 1, on agite puis laisse le mélange au repos pendant 1 heure, on recueille la phase organique et on la soumet à l'évaporation ou à une purification, de façon connue, par chromatographie sur colonne.

6. Composition selon l'une quelconque des revendications précédentes, caractérisée par la fait qu'elle contient comme ingrédient actif au moins un glycopeptidolipide de formule 1 : 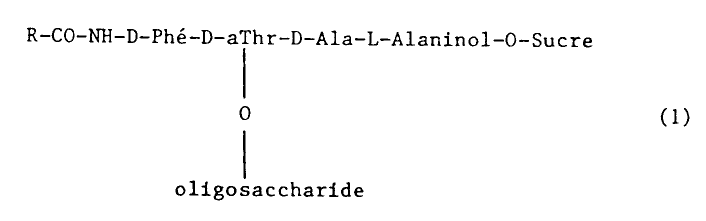 où Phé représente la phénylalanine,
aThr représente l'allo-thréonine,
Ala représente l'alanine,
le sucre est le 3,4-di-O-méthyl rhamnose,
l'oligosaccharide a la structure suivante :
3,4-di-O-méthylrhamnose-rhamnose-6-désoxytalose,
R-CO- est le reste acyle d'un acide gras,
les groupements 3,4-di-O-méthylrhamnose étant reliés respectivement à l'allo-thréonine et à l'alaninol par une liaison glycosidique,
et le groupement C terminal de l'alanine étant lié à l'alaninol par une liaison amide.

7. Composition selon la revendication 6, caractérisée par le fait que R-CO- est le reste acyle d'un acide gras ayant au moins 16 atomes de carbone.

8. Composition selon la revendication 7, caractérisée par le fait que R-CO- est le reste acyle d'un acide gras ayant moins de 36 atomes de carbone.

9. Composition selon la revendication 7 ou 8, caractérisée par la fait que ledit acide gras contient une ou plusieurs doubles liaisons et/ou une ramification et/ou un substituant bêta-hydroxy ou bêta-méthoxy.

10. Composition selon l'une quelconque des revendications précédentes, caractérisée par le fait que ledit dérivé est un dérivé désacétylé.

11. Composition selon l'une quelconque des revendications 6 à 10, caractérisée par le fait que ledit ingrédient actif est au moins un composé de formule 1 obtenu par synthèse ou hémisynthèse.

12. Composition selon l'une quelconque des revendications précédentes, caractérisée par le fait qu'elle se présente sous la forme de gélules, de comprimés, de poudres, de suppositoires, de pâtes gingivales, de crèmes, ou de compositions conditionnées pour l'application nasale ou conjonctivale.

13. Composition selon l'une quelconque des revendications 1 à 11, caractérisée par le fait qu'elle se présente sous la forme d'une suspension dans un véhicule pharmaceutique liquide, ou d'un lyophilisat.

14. Composition selon l'une quelconque des revendications précédentes, caractérisée par le fait qu'elle contient en outre une quantité efficace d'un agent conservateur et/ou d'un agent tensio-actif.

15. Composition selon l'une quelconque des revendications précédentes, caractérisée par le fait qu'elle contient 0,02 à 8 % en poids d'ingrédient actif, par rapport au poids total de la composition.

16. Utilisation d'une fraction au moins partiellement purifiée de GPLp de M.chelonae, telle que définie dans l'une quelconque des revendications 1 à 11, comme ingrédient actif dans la préparation d'un médicament immunostimulant non spécifique ou d'un médicament anabolisant, comme adjuvant dans la réalisation d'un vaccin ou comme additif dans la préparation d'une composition alimentaire.

17. Utilisation selon la revendication 16, dans la préparation d'un médicament destiné à corriger les effets immunodépresseurs des thérapeutiques anti-cancéreuses.

## Revendications (Revendications pour l'(les) Etat(s) contractant(s) suivant(s): ES, GR)

1. Utilisation d'une fraction d'extraction au moins partiellement purifiée de glycopeptidolipides (GPL) polaires de Mycobacterium chelonae (M.chelonae) ou d'un dérivé, actif comme immunostimulant, de GPL polaires de M.chelonae, comme ingrédient actif dans la préparation d'un médicament immunostimulant non spécifique ou d'un médicament anabolisant, comme adjuvant dans la réalisation d'un vaccin ou comme additif dans la préparation d'une composition alimentaire.

2. Utilisation selon la revendication 1, dans la préparation d'un médicament destiné à corriger les effets immunodépresseurs des thérapeutiques anti-cancéreuses

3. Utilisation selon la revendication 1 ou 2, caractérisée par le fait que ladite fraction au moins partiellement purifiée est une fraction soluble dans le méthanol à + 4°C.

4. Utilisation selon la revendication 1 ou 2, caractérisée par le fait que ladite fraction est une fraction qui peut être obtenue par extraction de cellules ou de parois de M.chelonae à l'aide d'un mélange chloroforme : méthanol (2 : 1) à une température de 18 à 50 °C, et qui en outre est soluble dans le méthanol à 4 °C.

5. Utilisation selon la revendication 4, caractérisée par le fait que ladite fraction peut être obtenue par extraction à l'aide d'un mélange chloroforme : méthanol 2 : 1, puis addition de méthanol, refroidi à 4°C, en quantité suffisante pour que le rapport méthanol : chloroforme, en volume, soit au moins égal à 5 : 1, et recueil de la fraction restant en solution.

6. Utilisation selon la revendication 5, caractérisée par le fait qu'en outre ladite fraction restant en solution est reprise, après évaporation des solvants, dans un mélange chloroforme : méthanol 2 : 1, est soumise, de façon connue, à une désacétylation par addition d'une solution 0,2 M de NaOH dans le méthanol, et qu'après 30 minutes à 37°C, on neutralise avec de l' acide acétique, on ajuste les proportions de solvants pour obtenir un mélange chloroforme : méthanol : eau à 4 : 2 : 1, on agite puis laisse le mélange au repos pendant 1 heure, on recueille la phase organique et on la soumet à l'évaporation ou à une purification, de façon connue, par chromatographie sur colonne.

7. Utilisation selon l'une quelconque des revendications précédentes, caractérisée par la fait que ledit ingrédient actif contient au moins un glycopeptidolipide de formule 1 : 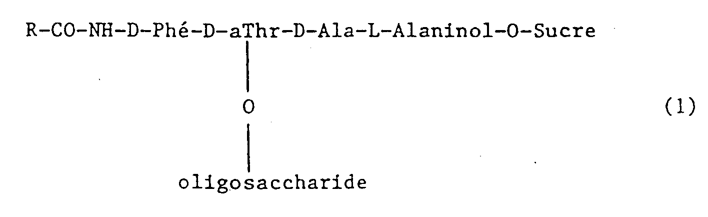 où Phé représente la phénylalanine,
aThr représente l'allo-thréonine,
Ala représente l'alanine,
le sucre est le 3,4-di-O-méthyl rhamnose,
l'oligosaccharide a la structure suivante :
3,4-di-O-méthylrhamnose-rhamnose-6-désoxytalose,
R-CO- est le reste acyle d'un acide gras,
les groupements 3,4-di-O-méthylrhamnose étant reliés respectivement à l'allo-thréonine et à l'alaninol par une liaison glycosidique,
et le groupement C terminal de l'alanine étant lié à l'alaninol par une liaison amide.

8. Utilisation selon la revendication 7, caractérisée par le fait que R-CO- est le reste acyle d'un acide gras ayant au moins 16 atomes de carbone.

9. Utilisation selon la revendication 8, caractérisée par le fait que R-CO- est le reste acyle d'un acide gras ayant moins de 36 atomes de carbone.

10. Utilisation selon la revendication 8 ou 9, caractérisée par la fait que ledit acide gras contient une ou plusieurs doubles liaisons et/ou une ramification et/ou un substituant bêta-hydroxy ou bêta-méthoxy.

11. Utilisation selon l'une quelconque des revendications précédentes, caractérisée par le fait que ledit dérivé est un dérivé désacétylé.

12. Utilisation selon l'une quelconque des revendications 7 à 11, caractérisée par le fait que ledit ingrédient actif est au moins un composé de formule 1 obtenu par synthèse ou hémisynthèse.

13. Utilisation selon l'une quelconque des revendications précédentes, caractérisée par le fait que ladite composition se présente sous la forme de gélules, de comprimés, de poudres, de suppositoires, de pâtes gingivales, de crèmes, ou de compositions conditionnées pour l'application nasale ou conjonctivale.

14. Utilisation selon l'une quelconque des revendications 1 à 12, caractérisée par le fait que ladite composition se présente sous la forme d'une suspension dans un véhicule pharmaceutique liquide, ou d'un lyophilisat.

15. Utilisation selon l'une quelconque des revendications précédentes, caractérisée par le fait que ladite composition contient en outre une quantité efficace d'un agent conservateur et/ou d'un agent tensio-actif.

16. Utilisation selon l'une quelconque des revendications précédentes, caractérisée par le fait que ladite composition contient 0,02 à 8 % en poids d'ingrédient actif, par rapport au poids total de la composition.

## Claims (Claims for the following Contracting State(s): AT, BE, CH, DE, DK, FR, GB, IT, LI, LU, NL, SE)

1. Pharmaceutical composition, characterized in that it contains as active ingredient an at least partially purified extraction fraction of Mycobacterium chelonae (M.chelonae) polar glycopeptidolipids (GPL), or of a derivative of M.chelonae polar GPLs which is active as an immunostimulant.

2. Composition according to Claim 1, characterized in that the said at least partially purified fraction is a fraction soluble in methanol at +4°C.

3. Composition according to Claim 1, characterized in that the said fraction is a fraction which can be obtained by extraction of M.chelonae cells or walls using a chloroform/methanol (2:1) mixture at a temperature of 18 to 50°C and which, in addition, in soluble in methanol at 4°C.

4. Composition according to Claim 3, characterized in that the said fraction can be obtained by extraction using a 2:1 chloroform/methanol mixture, followed by addition of methanol cooled to 4°C in a sufficient amount for the methanol/chloroform ratio by volume to be equal to at least 5:1, and collection of the fraction remaining in solution.

5. Composition according to Claim 4, characterized in that, in addition, the said fraction remaining in solution is taken up after the solvents are evaporated off in a 2:1 chloroform/methanol mixture and is subjected in a known manner to a deacetylation by adding a 0.2M solution of NaOH in methanol, and in that, after 30 minutes at 37°C, the mixture is neutralized with acetic acid, the proportions of solvents are adjusted to obtain a chloroform/methanol/water mixture in the ratios 4:2:1, the mixture is stirred and then left standing for 1 hour, and the organic phase is collected and subjected to evaporation or to a purification in a known manner by column chromatography.

6. Composition according to any one of the preceding claims, characterized in that it contains as active ingredient at least one glycopeptidolipid of formula 1: 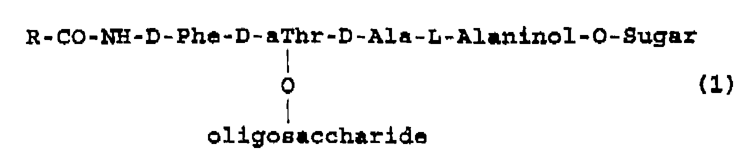 where Phe represents phenylalanine,
aThr represents allothreonine,
Ala represents alanine,
the sugar is 3,4-di-O-methylrhamnose,
the oligosaccharide has the following structure:
3,4-di-O-methylrhamnose-rhamnose-6-deoxytalose,
R-CO- is the acyl residue of a fatty acid,
the 3,4-di-O-methylrhamnose groups being joined, respectively, to the allothreonine and to the alaninol via a glycosidic bond,
and the C-terminal group of the alanine being linked to the alaninol via an amide bond.

7. Composition according to Claim 6, characterized in that R-CO- is the acyl residue of a fatty acid having at least 16 carbon atoms.

8. Composition according to claim 7, characterized in that R-CO- is the acyl residue of a fatty acid having fewer than 36 carbon atoms.

9. Composition according to Claim 7 or 8, characterized in that the said fatty acid contains one or more double bonds and/or a side chain and/or a beta-hydroxy or beta-methoxy substituent.

10. Composition according to any one of the preceding claims, characterized in that the said derivative is a deacetylated derivative.

11. Composition according to any one of Claims 6 to 10, characterized in that the said active ingredient is at least one compound of formula 1 obtained by synthesis or partial synthesis.

12. Composition according to any one of the preceding claims, characterized in that it takes the form of hard gelatin capsules, tablets, powders, suppositories, gum ointments, creams or compositions packaged for nasal or conjunctival application.

13. Composition according to any one of Claims 1 to 11, characterized in that it takes the form of a suspension in a liquid pharmaceutical vehicle, or of a lyophilisate.

14. Composition according to any one of the preceding claims, characterized in that it contains, in addition, an effective amount of a preservative and/or of a surfactant.

15. Composition according to any one of the preceding claims, characterized in that it contains 0.02 to 8% by weight of active ingredient relative to the total weight of the composition.

16. Use of an at least partially purified fraction of M.chelonae pGPL as is defined in any one of Claims 1 to 11, as active ingredient in the preparation of a non-specific immunostimulatory medicinal product or of an anabolic medicinal product, as adjuvant in the production of a vaccine or as additive in the preparation of a food composition.

17. Use according to Claim 16, in the preparation of a medicinal product intended for correcting the immunosuppressant effects of anticancer therapies.

## Claims (Claims for the following Contracting State(s): ES, GR)

1. Use of an at least partially purified extraction fraction of Mycobacterium chelonae (M.chelonae) polar glycopeptidolipids (GPL), or of a derivative of M.chelonae polar GPLs which is active as an immunostimulant, as active ingredient in the preparation of a non-specific immunostimulatory medicinal product or of an anabolic medicinal product, as adjuvant in the production of a vaccine or as additive in the preparation of a food composition.

2. Use according to Claim 1, in the preparation of a medicinal product intended for correcting the immunosuppressant effects of anticancer therapies.

3. Use according to Claim 1 or 2, characterized in that the said at least partially purified fraction is a fraction soluble in methanol at +4°C.

4. Use according to Claim 1 or 2, characterized in that the said fraction is a fraction which can be obtained by extraction of M.chelonae cells or walls using a chloroform/methanol (2:1) mixture at a temperature of 18 to 50°C and which, in addition, is soluble in methanol at 4°C.

5. Use according to Claim 4, characterized in that, the said fraction can be obtained by extraction using a 2:1 chloroform/methanol mixture, followed by addition of methanol cooled to 4°C in a sufficient amount for the methanol/chloroform ratio by volume to be equal to at least 5:1, and collection of the fraction remaining in solution.

6. Use according to Claim 5, characterized in that, in addition, the said fraction remaining in solution is taken up after the solvents are evaporated off in a 2:1 chloroform/methanol mixture and is subjected in a known manner to a deacetylation by adding a 0.2M solution of NaOH in methanol, and in that, after 30 minutes at 37°C, the mixture is neutralized with acetic acid, the proportions of solvents are adjusted to obtain a chloroform/methanol/water mixture in the ratios 4:2:1, the mixture is stirred and then left standing for 1 hour, and the organic phase is collected and subjected to evaporation or to a purification in a known manner by column chromatography.

7. Use according to any one of the preceding claims, characterized in that the said active ingredient contains at least one glycopeptidolipid of formula 1: 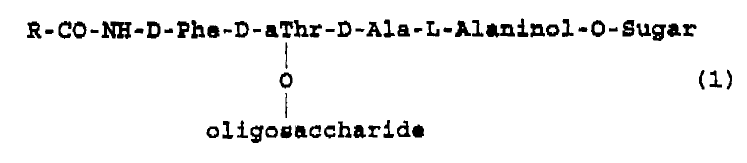 where Phe represents phenylalanine,
aThr represents allothreonine,
Ala represents alanine,
the sugar is 3,4-di-O-methylrhamnose,
the oligosaccharide has the following structure:
3,4-di-O-methylrhamnose-rhamnose-6-deoxytalose,
R-CO- is the acyl residue of a fatty acid,
the 3,4-di-O-methylrhamnose groups being joined, respectively, to the allothreonine and to the alaninol via a glycosidic bond,
and the C-terminal group of the alanine being linked to the alaninol via an amide bond.

8. Use according to Claim 7, characterized in that R-CO- is the acyl residue of a fatty acid having at least 16 carbon atoms.

9. Use according to Claim 8, characterized in that R-CO- is the acyl residue of a fatty acid having fewer than 36 carbon atoms.

10. Use according to Claim 8 or 9, characterized in that the said fatty acid contains one or more double bonds and/or a side chain and/or a beta-hydroxy or beta-methoxy substituent.

11. Use according to any one of the preceding claims, characterized in that the said derivative is a deacetylated derivative.

12. Use according to any one of Claims 7 to 11, characterized in that the said active ingredient is at least one compound of formula 1 obtained by synthesis or partial synthesis.

13. Use according to any one of the preceding claims, characterized in that the said composition takes the form of hard gelatin capsules, tablets, powders, suppositories, gum ointments, creams or compositions packaged for nasal or conjunctival application.

14. Use according to any one of Claims 1 to 12, characterized in that the said composition takes the form of a suspension in a liquid pharmaceutical vehicle, or of a lyophilisate.

15. Use according to any one of the preceding claims, characterized in that the said composition contains, in addition, an effective amount of a preservative and/or of a surfactant.

16. Use according to any one of the preceding claims, characterized in that the said composition contains 0.02 to 8% by weight of active ingredient relative to the total weight of the composition.

## Patentansprüche (Patentansprüche für folgende(n) Vertragsstaat(en): AT, BE, CH, DE, DK, FR, GB, IT, LI, LU, NL, SE)

1. Arzneimittel, dadurch gekennzeichnet, daß es als aktiven Wirkstoff eine mindestens partiell gereinigte Extraktionsfraktion von polaren Glycopeptidolipiden (GPL) aus Mycobakterium chelonae (M.chelonae) oder ein als Immunstimulans wirkendes Derivat von polaren Glycopeptidolipiden von M.chelonae enthält.

2. Mittel gemäß Anspruch 1, dadurch gekennzeichnet, daß die mindestens partiell gereinigte Fraktion eine bei +4 °C in Methanol lösliche Fraktion darstellt.

3. Mittel gemäß Anspruch 1, dadurch gekennzeichnet, daß die Fraktion eine Fraktion ist, die mittels der Extraktion von Zellen oder Zellwänden von M.chelonae mit einem Gemisch aus Chloroform und Methanol (2:1) bei einer Temperatur von 18 bis 50 °C erhalten werden kann und die bei 4 °C in Methanol löslich ist.

4. Mittel gemäß Anspruch 3, dadurch gekennzeichnet, daß die Fraktion durch Extraktion mit einem Gemisch aus Chloroform und Methanol (2:1) erhalten wird, gefolgt von der Zugabe von Methanol, nach Abkühlen auf 4 °C, in ausreichender Menge, damit das Volumenverhältnis von Methanol : Chloroform mindestens oder gleich 5:1 beträgt und die in Lösung verbleibende Fraktion gewonnen wird.

5. Mittel gemäß Anspruch 4, dadurch gekennzeichnet, daß die in Lösung verbleibende Fraktion darüber hinaus, nach dem Abdampfen der Lösungsmittel, wieder in einem Gemisch aus Chloroform und Methanol (2:1) aufgenommen und auf bekannte Weise der Verseifung (Desacetylierung) unterzogen wird, indem man eine 0,2 M NaOH-Lösung in Methanol zugibt und nach 30 min bei 37 °C mit Essigsäure neutralisiert, die Lösungsmittelanteile zum Erhalt einer Mischung aus Chloroform, Methanol und Wasser (4:2:1) einstellt, verrührt und das Gemisch 1 Stunde lang stehenläßt, die organische Phase abnimmt und sie abdampft oder einer auf bekannte Weise erfolgten Reinigung mittels Säulenchromatographie unterzieht.

6. Mittel gemäß einem der vorstehenden Ansprüche, dadurch gekennzeichnet, daß es als aktiven Wirkstoff mindestens ein Glycopeptidolipid der Formel 1 enthält: 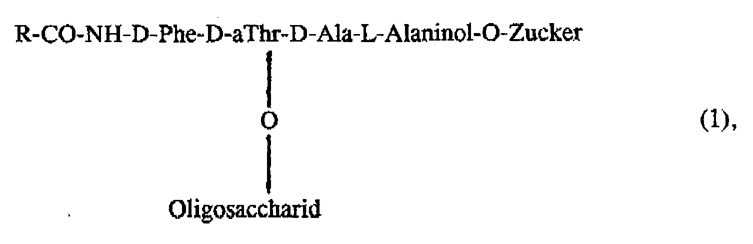 in der
Phe Phenylalanin,
aThr Allothreonin und
Ala Alanin bedeuten,
der Zucker 3,4-Di-O-methylrhamnose ist,
das Oligosaccharid die folgende Struktur hat:
3,4-Di-O-methylrhamnose-Rhamnose-6-Desoxytalose,
R-CO einen Fettsäureacylrest darstellt,
die 3,4-Di-O-methylrhamnosegruppen jeweils mittels glycosidischer Bindungen an das Allothreonin bzw. das Alaninol gebunden sind und
das C-terminale Ende des Alanins über eine Amidbindung mit dem Alaninol verbunden ist.

7. Mittel gemäß Anspruch 6, dadurch gekennzeichnet, daß der Rest R-CO- einen Fettsäureacylrest mit mindestens 16 Kohlenstoffatomen darstellt.

8. Mittel gemäß Anspruch 7, dadurch gekennzeichnet, daß der REst R-CO- einen Fettsäureacylrest mit mindestens 36 Kohlenstoffatomen darstellt.

9. Mittel gemäß einem der Ansprüche 7 oder 8, dadurch gekennzeichnet, daß die Fettsäure eine oder mehrere Doppelbindungen und/oder Verzweigungen und/oder einen β-Hydroxy- oder β-Methoxysubstituenten aufweist.

10. Mittel gemäß einem der vorstehenden Ansprüche, dadurch gekennzeichnet, daß das Derivat einen desacetylierten Abkömmling darstellt.

11. Mittel gemäß einem der Ansprüche 6 bis 10, dadurch gekennzeichnet, daß der aktive Wirkstoff mindestens eine mittels Synthese oder Halbsynthese erhaltene Verbindung der Formel 1 darstellt.

12. Mittel gemäß einem der vorstehenden Ansprüche, dadurch gekennzeichnet, daß sie in Form von Kapseln, Tabletten, Pulvern, Zäpfchen, Zahnfleischpastern, Cremes oder für die nasale oder Bindehautapplikation hergerichteten Mitteln vorliegt.

13. Mittel gemäß einem der Ansprüche 1 bis 11, dadurch gekennzeichnet, daß es in Form einer Suspension in einem flüssigen, pharmazeutischen Träger oder in Form eines Lyophilisates vorliegt.

14. Mittel gemäß einem der vorstehenden Ansprüche, dadurch gekennzeichnet, daß es zusätzlich eine wirksame Menge eines Konservierungsmittels und/oder eines oberflächenaktiven Mittels enthält.

15. Mittel gemäß einem der vorstehenden Ansprüche, dadurch gekennzeichnet, daß es, bezogen auf das Gesamtgewicht der Mittel, 0,02 bis 8 Gew.-% des aktiven Wirkstoffes enthält.

16. Verwendung einer mindestens teilweise gereinigten Glycopeptidolipidfraktion aus M.chelonae gemäß einem der Ansprüche 1 bis 11 als aktiver Wirkstoff in einem unspezifischen Immunstimulans oder einem Anabolikum als Adjuvans für ein Vakzin oder als Additiv bei der Herstellung eines Lebensmittels.

17. Verwendung gemäß Anspruch 16 bei der Herstellung eines Medikamentes, welches zur Korrektur von immundepressiven Effekten bei der Krebstherapie bestimmt ist.

## Patentansprüche (Patentansprüche für folgende(n) Vertragsstaat(en): ES, GR)

1. Verwendung einer mindestens partiell gereinigten Extraktionsfraktion von polaren Glycopeptidolipiden (GPL) aus Mycobakterium chelonae (M.chelonae) oder eines als Immunostimulans wirkenden Derivats von polaren Glycopeptidolipiden von M.chelonae als aktiven Wirkstoff in einer Mittel eines unspezifischen Immunostimulans oder eines Anabolikums als Adjuvans für ein Vakzin oder als Additiv bei der Herstellung eines Lebensmittels.

2. Verwendung gemäß Anspruch 1 bei der Herstellung eines Medikamentes, welches zur Korrektur von immunodepressiven Effekten bei der Krebstherapie bestimmt ist.

3. Verwendung gemäß Anspruch 1 oder 2, durch gekennzeichnet, daß die mindestens partiell gereinigte Fraktion eine bei +4 °C in Methanol lösliche Fraktion darstellt.

4. Verwendung gemäß Anspruch 1 oder 2, dadurch gekennzeichnet, daß die Fraktion eine Fraktion darstellt, die mittels der Extraktion von M.chelonae Zellen oder Zellwänden mit einem Gemisch aus Chloroform und Methanol (2:1) bei einer Temperatur von 18 bis 50 °C erhalten werden kann und die bei 4 °C in Methanol löslich ist.

5. Verwendung gemäß Anspruch 4, dadurch gekennzeichnet, daß die Fraktion durch Extraktion mit einem Gemisch aus Chloroform und Methanol (2:1) erhalten wird, gefolgt von der Zugabe von Methanol, nach Abkühlen auf 4 °C, in ausreichender Menge, damit das Volumenverhältnis von Methanol : Chloroform mindestens oder gleich 5:1 beträgt und die in Lösung verbleibende Fraktion gewonnen wird.

6. Verwendung gemäß Anspruch 5, dadurch gekennzeichnet, daß die in Lösung verbleibende Fraktion darüber hinaus, nach dem Abdampfen der Lösungsmittel, wieder in einem Gemisch aus Chloroform und Methanol (2:1) aufgenommen und auf bekannte Weise der Verseifung (Desacetylierung) unterzogen wird, indem man eine 0,2 M NaOH-Lösung in Methanol zugibt und nach 30 min bei 37 °C mit Essigsäure neutralisiert, die Lösungsmittelanteile zum Erhalt einer Mischung aus Chloroform, Methanol und Wasser (4:2:1) einstellt, verrührt und das Gemisch 1 Stunde stehenläßt, die organische Phase abnimmt und sie abdampft oder einer auf bekannte Weise erfolgten Reinigung mittels Säulenchromatographie unterzieht.

7. Verwendung gemäß einem der vorstehenden Ansprüche, dadurch gekennzeichnet, daß der aktive Wirkstoff mindestens ein Glycopeptidolipid der Formel 1 umfaßt: 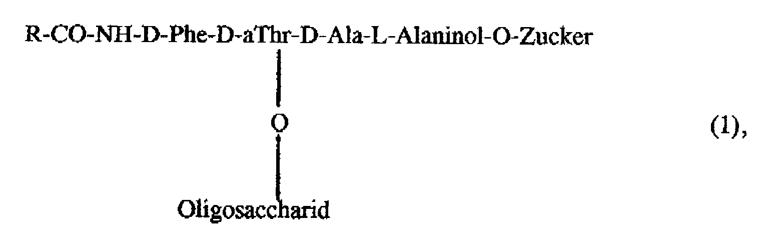 in der
Phe Phenylalanin,
aThr Allothreonin und
Ala Alanin bedeuten,
der Zucker 3,4-Di-O-methylrhamnose ist,
das Oligosaccharid die folgende Struktur hat:
3,4-Di-O-methylrhamnose-Rhamnose-6-Desoxytalose,
R-CO einen Fettsäureacylrest darstellt,
die 3,4-Di-O-methylrhamnosegruppen jeweils mittels glycosidischer Bindungen an das Allothreonin bzw. das Alaninol gebunden sind und das C-terminale Ende des Alanins über eine Amidbindung mit dem Alaninol verbunden ist.

8. Verwendung gemäß Anspruch 7, dadurch gekennzeichnet, daß der Rest R-CO- einen Fettsäureacylrest mit mindestens 16 Kohlenstoffatomen darstellt.

9. Verwendung gemäß Anspruch 8, dadurch gekennzeichnet, daß der Rest R-CO- einen Fettsäureacylrest mit mindestens 36 Kohlenstoffatomen darstellt.

10. Verwendung gemäß einem der Ansprüche 8 oder 9, dadurch gekennzeichnet, daß die Fettsäure eine oder mehrere Doppelbindungen und/oder Verzweigungen und/oder einen β-Hydroxy- oder β-Methoxysubstituenten aufweist.

11. Verwendung gemäß einem der vorstehenden Ansprüche, dadurch gekennzeichnet, daß das Derivat ein desacetyliertes Derivat darstellt.

12. Verwendung gemäß einem der Ansprüche 7 bis 11, dadurch gekennzeichnet, daß der aktive Wirkstoff mindestens eine mittels Synthese oder Halbsynthese erhaltene Verbindung der Formel 1 darstellt.

13. Verwendung gemäß einem der vorstehenden Ansprüche, dadurch gekennzeichnet, daß das Mittel in Form von Kapseln, Tabletten, Pulvern, Zäpfchen, Zahnfleischpasten, Cremes oder für die nasale oder Bindehautapplikation hergerichteten Mitteln vorliegt.

14. Verwendung gemäß einem der Ansprüche 1 bis 12, dadurch gekennzeichnet, daß das Mittel in Form einer Suspension in einem flüssigen, pharmazeutischen Träger oder in Form eines Lyophilisates vorliegt.

15. Verwendung gemäß einem der vorstehenden Ansprüche, dadurch gekennzeichnet, daß das Mittel zusätzlich eine wirksame Menge eines Konservierungsmittels und/oder eines oberflächenaktiven Mittels enthält.

16. Verwendung gemäß einem der vorstehenden Ansprüche, dadurch gekennzeichnet, daß das Mittel 0,02 bis 8 Gew.-% des aktiven Wirkstoffes, bezogen auf das Gesamtgewicht der Mittel, enthält.
